# EUROPEAN PATENT APPLICATION

(11) **EP 1 469 312 A1**
(43) Date of publication of application: **20.10.2004**
(21) Application number: 03009070.8
(22) Date of filing: 18.04.2003
(51) Int. Cl.: G01N 33/68

(54) **Diagnosis of Alzheimer's disease**

(71) Applicant: Friedrich-Alexander-Universität Erlangen-Nürnberg, 91054 Erlangen (DE)
(72) Inventor: Wiltfang, Jens, 91056 Erlangen (DE); Lewczuk, Piotr, 91058 Erlangen (DE); Kornhuber, Johannes, 90491 Nürnberg (DE)
(74) Representative: Patentanwälte Rehberg + Hüppe

(57) **Abstract**

A method of diagnosis of Alzheimer's disease comprises the step of determining an amount of at least one species in a body fluid or a tissue homogenate sample which has been obtained from a patient. The at least one species is selected from a group of species having molecular masses in a range of 3870 to 3895 Da (Abeta 1-34), 4520 to 4545 (Abeta 3-44), 4802 to 4836 Da (Abeta 2-46 or Abeta 1-45), 4837 to 4871 Da (Abeta 3-47) and 7720 to 7790 Da, respectively.

## Description

### FIELD OF THE INVENTION

The present invention generally relates to the diagnosis of Alzheimer's disease. More particular, the invention relates to a method of diagnosis of Alzheimer's disease comprising the step of determining an amount of certain species in a body fluid or a tissue homogenate sample which has been obtained from a patient.

### BACKGROUND OF THE INVENTION

From EP 1 270 592 A1 it is known that a diagnosis of Alzheimer's disease is possible by determining the amounts of certain Amyloid-beta peptides in a body fluid or a tissue homogenate sample which has been obtained from a patient. Particular reference is made to the Aβ-peptides Aβ1-42, Aβ2-42, Aβ1-40, Aβ1-38, Aβ1-37 und Aβ1-39. Amounts and/or quotients of amounts of certain of these Aβ-peptides show characteristic changes with the occurrence of Alzheimer's disease. For example, the amount of Aβ1-42 is typically decrease in case of Alzheimer's disease whereas the amount of Aβ1-40 remains unchanged. Thus, a decrease of the quotient Aβ1-42/Aβ1-40 below a cut off value is a good indication of the presence of Alzheimer's disease, and it is independent of fluctuations in the absolute amount or concentrations of these Aβ peptides, which may be caused not only by individual variations but also by variations in obtaining and processing the samples from the individuals.

According to the disclosure of EP 1 270 592 A1 the Aβ-peptides are first collected or concentrated from the samples by means of an antibody directed against Aβ-peptides. Particularly a monoclonal antibody which is referred to as mAb 1E8 and which is specific for the first two N-terminal amino acids of Amyloid-beta peptides is used. Afterwards the Aβ-peptides are separated from one another by a sodium lauryl sulfate polyacrylamide electrophoresis (SDS-PAGE). The separation may be further enhanced by isoelectric focusing the Aβ peptides.

From Merchand and Weinberger, 2000 (see reference (13) for the second study of the detailed description) it is known how to determine the molecular masses of molecule species contained in a sample. This method is also referred to by its abbreviation SELDI-TOF-MS. In the method, the species of interest are fixed to a specified surface, for example by means of an anion exchanger or an antibody which is itself covalently bond to the surface. Then, the species are desorbed and ionized by means of a laser beam focused onto the surface. This ionization involves the matrix to which the species of interest are bond. The desorbed and ionized species are accelerated by a static electrical high voltage field. The period of time between the desorption/ionization of the species from the surface and the activation of a detector (= time of flight) is directly proportional to the square root of the mass of the respective species. The mass of proteins having a molecular mass in the range of a few thousand Da may be measured with an accuracy better than 0.1 %.

ProteinChip is the trademark of a system for SELDI-TOF-MS which is commercially available from Ciphergen Biosystems Inc.

### PROBLEM OF THE INVENTION

The problem to be solved by the present invention is to disclose further species which may be found in body fluid or tissue homogenate samples obtained from a patient and which are indicative of the presence or non-presence of Alzheimer's disease. These additional species would be useful even if they allow no better discrimination of non Alzheimer's disease patients from Alzheimer's disease patients, because they would provide a further opportunity for this discrimination, which is an advantage as such. Of course, species which allow an even better discrimination than the Aβ-peptides known from EP 1 270 592 A1 would be particularly desirable.

### SUMMARY OF THE INVENTION

The invention provides a method of diagnosis of Alzheimer's disease according to claim 1. Preferred embodiments of the new method are disclosed in the dependent claims 2 to 18.

In one aspect of the invention five new species indicative of the presence or non-presence of Alzheimer's disease are disclosed by their molecular masses falling in certain ranges. These new species have been discovered using SELDI-TOF-MS. However, any other method allowing an analysis of molecular masses can also be applied.

Vice versa it is no limiting feature of the intention that the new species may be identified by their molecular masses. Most of them have been clearly or most probably identified as certain Aβ-peptides. If the amounts of these Aβ-peptides are determined in another way than by their molecular masses this is also an application of the method of the present invention.

With regard to the species having the molecular mass in the range of 4802 to 4836 Da it is most probably that it is the Amyloid-beta peptide Aβ2-46. However, with regard to the observed mass it can not yet be excluded that this species is Aβ1-45. The expected molecular masses of Aβ2-46 and Aβ1-45 are 4811.62 and 4827.49, respectively.

According to the investigations which will be reported in detail in the following, the presence of the species having the molecular mass in the range of 4802 to 4846 Da may be regarded as an indication of the presence of Alzheimer's disease, because both in case of cerebrospinal fluid and brain tissue pools of Alzheimer's disease patients and non Alzheimer's disease controls this species was only found in the Alzheimer's disease pools.

The species having the molecular mass in the range of 4520 to 4545 Da is considered as being Aβ3-44 having an expected molecular mass of 4528.2 Da.

The species having the molecular mass in the range of 4837 to 4871 Da is considered as being Aβ3-47 having an expected molecular mass of 4853.6 Da.

The investigations reported in the following demonstrate that a quotient of the amounts of the species having the molecular masses in the ranges of 4520 to 4545 Da and 4837 to 4871 Da can advantageously be used for diagnoses of Alzheimer's disease, because in case of an Alzheimer's disease the amount of the species having the molecular mass in the range of 4520 to 4545 Da decreases whereas the amount of the species having the molecular mass in the range of 4837 to 4871 Da increases. If, for example, the amounts of the species are measured by SELDI-TOF-MS as signal to noise ratios, a cut off value of about 0.95 can discriminate the patients with Alzheimer's disease who have a quotient below this cut off value from the non Alzheimer's disease controls with a specificity of about 90 % and a sensitivity of 100 %. This cut off value may be modified by further investigations but its uncertainty is not higher than 30 %. Other procedures used for measuring the amounts of these two species may however further alter the cut off value but an appropriate cut off value is easily set for any amount measuring method.

The species having the molecular mass in the range of 3870 to 3895 Da is not considered as really having this molecular mass but as being a molecule which has been double charged in SELDI-TOF-MS so that it only shows half of its real molecular mass which is indeed within the range of 7720 to 7790.00 DA.

The amount of the species having the molecular mass in the range of 7720 to 7790 Da decreases in case of an Alzheimer's decrease. Thus, a decrease of this amount below a cut off value may be regarded as an indication of the presence of Alzheimer's disease.

With regard to signal to noise ratios obtained from SELDI-TOF-MS an appropriate cut off value is 6.1. For the same reasons as above, this value may have to be modified but it will still be in the range of 4 to 7.8 as long as the signal to noise ratios are obtained by SELDI-TOF-MS with an appropriate setting of the region of interest.

All cut off values indicated here have been obtained from evaluations of cerebrospinal fluid and brain tissue samples, only. Thus, the particular values are only valid for these kinds of samples.

In a preferred embodiment of the new method the species of interest are concentrated from the respective sample in that the sample is exposed to an antibody directed against Amyloid-beta peptides prior to determining the amount of the species of interest in a fraction of the sample bond to the antibody. If SELDI-TOF-MS is used as the method for determining the amount of the species of interest, the antibody may also be used to fix the species of interest to the surface at which they are ionised.

Using SELDI-TOF-MS it could be confirmed that two further species having molecular masses in the ranges of 4510 to 4519 Da and 4320 to 4340 Da, respectively, may be used in the diagnosis of Alzheimer's disease. These species are considered as being the Amyloid-beta peptides Aβ1-42 and Aβ1-40. It is already known that the level of Aβ1-42 decreases with the occurrence of Alzheimer's disease whereas the amount of Aβ1-40 remains constant. Thus, the quotient of the amounts of Aβ1-42 and Aβ1-40 is highly indicative of the presence of Alzheimer's disease. At the same time this quotient is insensitive to any variations in obtaining the respective samples and in further steps of the evaluation of the samples.

Using SELDI-TOF-MS a cut off value for the quotient of the signal to noise ratios of Aβ1-42/Aβ1-40 which is 0.175 and which may be further fine tuned within a range of 0,123 and 0,3 discriminates the non Alzheimer's disease patients from the Alzheimer's disease patients with both a specificity and sensitivity of about 80 %.

Thus, it has been shown that SELDI-TOF-MS, especially if combined with an antibody directed against Amyloid-beta peptides is a suitable means for the diagnosis of Alzheimer's disease, even if only the amounts of the known Aß-peptides Aβ1-42 and Aβ1-40 are determined and evaluated.

Those skilled in the art will easily recognize that there are further suitable quotients of amounts of the disclosed and further Aβ-peptides which are clearly indicative of the presence of Alzheimer's disease. As a general rule it is useful to have a quotient of the amount of a species which is varied by Alzheimer disease in one direction and an amount of a species which is varied by Alzheimer's disease in the other direction, or of an amount of a species which is changing with an Alzheimer's disease in any direction and an amount of a species which is not changing with an Alzheimer's disease. In any of these cases any methodical error which affects the amounts of both species by about the same error factor is compensated for.

Other features and advantages of the present invention will become apparent to one with skill in the art upon examination of the following Figures and the detailed description. It is intended that all such additional features and advantages be included herein within the scope of the present invention, as defined by the claims.

### BRIEF DESCRIPTION OF THE FIGURES

The invention can be better understood with reference to the accompanying Figures.

**Fig. 1** is a SELDI-TOF analysis of the patterns of Aβ peptides in human pooled CSF from patients with Alzheimer's disease and non-demented controls. The observed molecular masses are presented. The insert presents the spectral region with the peak corresponding to a novel carboxyterminally elongated peptide.
Fig. 1A Mass spectrometry analysis of the AD CSF incubated with bovine IgG;
Fig. 1B Mass spectrometry analysis of the control CSF incubated with bovine IgG;
Fig. 1C Analysis of the control CSF incubated with the antibody against Aβ peptides (6E10);
Fig. 1D Analysis of the AD CSF incubated with the antibody against Aβ peptides (6E10).

**Fig. 2** is an Aβ-SDS-PAGE/immunoblot of the pooled CSF. As the standard, a mixture of the peptides Aβ: 1-37, 1-38, 1-39, 1-40, 1-42 and 2-42 was applied.

**Fig. 3** is a SELDI-TOF analysis of the patterns of Aβ peptides in the homogenates of human *post mortem* brain tissue from patients with Alzheimer's disease and non-demented controls. The observed molecular masses are presented. The insert presents the spectral region with the peak corresponding to a novel carboxyterminally elongated peptide.
Fig. 3A Analysis of the control brain tissue incubated with the antibody against Aβ peptides (6E10).
Fig. 3B Analysis of the AD brain tissue incubated with the antibody against Aβ peptides (6E10).

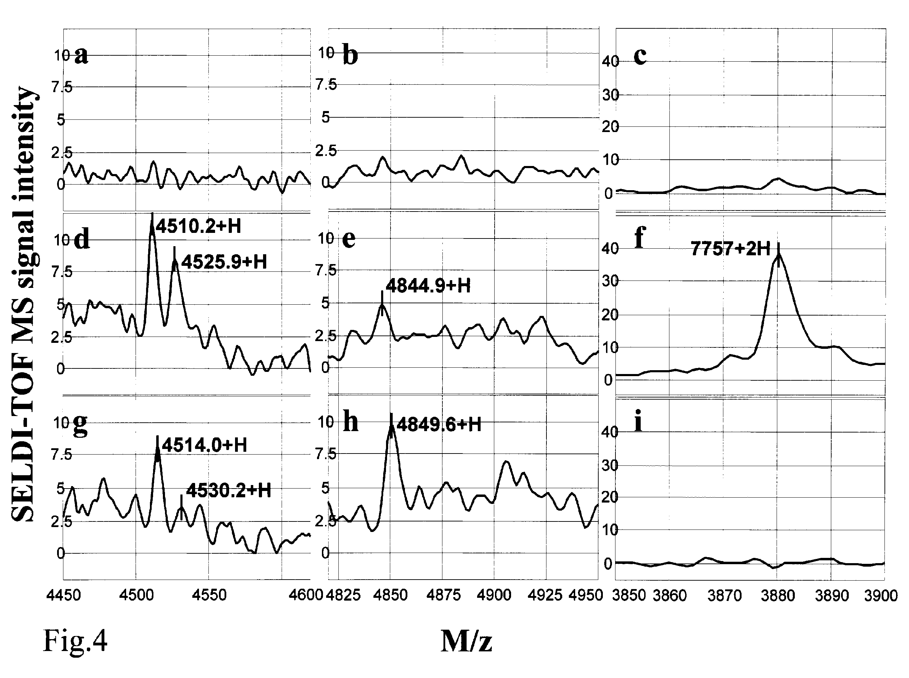

**Fig. 4** shows representative spectra of CSF from two subjects investigated in this study incubated either with unspecific bovine IgG or monoclonal antibody against Aβ peptides. Molecular masses measured in these patients are shown. In the Figures 4a, 4b and 4c, no significant signal is present in the investigated molecular mass range when CSF of a control subject was incubated with an array with bovine IgG. However, when CSF of this same subject was incubated with an array pre-activated with the antibody against Aβ peptides, 6E10, clear mass spectrometry signals were obtained (Figures 4d, 4e and 4f). Similarly, incubation of CSF from a patient with AD with an array with this same antibody evoked mass spectrometry signals (Figures 4g, 4h and 4i). M/z, the ratio of molecular mass to electric charge of an Aβ ion generated by SELDI.

**Fig. 5** shows signal-to-noise ratios of the investigated peptides. Open circles represent results of individual measurements. Horizontal bars represent medians.
Fig. 5a Signal-to-noise ratios of Aβ peptide with molecular mass 4525.1 Da;
Fig. 5b Signal-to-noise ratios of Aβ peptide with molecular mass 4846.8 Da;
Fig. 5c Signal-to-noise ratios of Aβ peptide with molecular mass 4512.0 Da, corresponding to Aβ1-42 peptide.
Fig. 5d Signal-to-noise ratios of Aβ peptide with molecular mass 4327.2 Da, corresponding to Aβ1-40 peptide.
Fig. 5e. Signal-to-noise ratios of a doubly charged Aβ peptide with molecular mass of 7755.8.

**Fig. 6** shows Quotients of the signal-to-noise ratios of the peptides investigated. Open circles represent individual results. Horizontal lines show cut off values best classifying the subjects studied.
Fig. 6a Quotient of two novel Aβ peptides separating correctly 18/19 (94.7%) of patients;
Fig. 6b Quotient of Aβ1-42/1-40 separating correctly 15/19 (79%) of patients.

### DETAILED DESCRIPTION

The detailed description of the invention comprises two main parts. Each of these main parts is related to one of two studies, which are described as embodiment examples of the claimed method. General background which applies to both studies and which is explained in the first study will not be repeated in full within the second study.

### First Study

### Overview

The patterns of Amyloid β (Aβ) peptides in human cerebrospinal fluid (CSF) and brain homogenates were studied by surface enhanced laser desorption/ionization (SELDI) time of flight (TOF) mass spectrometry (MS), and the results were compared to those obtained by Aβ-SDS-PAGE/immunoblot. Apart from the peptides known in the literature to occur in the CSF, the existence of a novel, previously not described peptide, either Aβ1-45 or Aβ2-46, is postulated. This peptide was observed exclusively in a pool of samples originating from patients with Alzheimer's disease (AD), i.e. cerebrospinal fluid (CSF) and *postmortem* brain homogenates, but neither in the pooled CSF samples nor the pooled brain homogenates of the non-demented controls. Similarly to previous results, Aβ1-42 was decreased in the CSF in AD. Expectedly, brain homogenates of the control subjects did not show the presence of Aβ-peptides. Compared to Aβ-SDS-PAGE/immunoblot, SELDI-TOF enabled more precise analysis of Aβ peptides in the human material. Thus, SELDI-TOF offers a promising tool for dementia expression pattern profiling in the minute amount of a biological sample.

### Introduction

Alzheimer's disease (AD) is the major cause of memory impairment and dementia in the elderly, and is one of the most severe causes of health problems in this group. Meanwhile, the advent of new therapeutic avenues calls for an improved early and differential diagnosis of AD. Since cerebrospinal fluid (CSF) is in direct contact with the central nervous system, several potentially promising CSF biomarkers have been tested alone or in combinations^{1,2}.

Among these biomarkers, Amyloid β peptides (Aβ peptides) fulfill the criteria for good AD diagnostic tests, as recently summarized by an expert review ³. This is not surprising since these peptides are directly involved in pathologic events of the disease, deposition of senile plaques. Aβ peptides are released from a transmembrane amyloid precursor protein (APP). Generation of Aβ peptides requires a common cut by two enzymes, β-, and γ-secretases whereas a cut by α-secretase results in a non-amyloidogenic metabolic pathway ⁴. The presence of several species of Aβ peptides in CSF and brain tissue implies enzymatic cuts at different positions of APP, and distinct γ-secretase activities are hypothesized ^{5,6}. In the scope of AD research, Aβ peptide ending at the amino acid position 42 (Aβ1-42) plays a central role since this neurotoxic factor is the main component of amyloid plaques. Aβ-42 CSF concentration is decreased in AD⁷⁻¹⁰, and this observation has already been incorporated into the neurochemical diagnosis of AD^{7, 8, 11, 12}. Due to alternative cutting positions of β- and γ-secretases, several other Aβ peptides have recently been described ⁸, ¹⁰, however, there may be even more undetected species of amyloid β peptides in human CSF.

Surface enhanced laser desorption/ionization (SELDI) technology is currently applied in many fields of biological and medical sciences, as extensively reviewed ¹³. It has recently been successfully used to analyze amyloid β peptides in supernatants of transfected HEK 293 cells ¹⁴ , however, the pattern of Aβ peptides has never been studied in human AD CSF with this method. Therefore, in this study SELDI-TOF was applied to analyze and compare the patterns of Aβ peptides in human pooled CSF from patients with Alzheimer's disease and non-demented controls. Further, the patterns of Aβ peptides in homogenates of the brain tissue obtained from patients with a definite neuropathological diagnosis of AD were compared with those without dementia.

### Materials and Methods

### 1. Cerebrospinal fluid and brain homogenates

In all cases, CSF was obtained for the purposes of routine diagnostic procedures by a spinal tap. CSF from ten patients diagnosed according to the generally accepted criteria of Alzheimer's disease, ICD-10, and National Institute of Neurological and Communicative Disorders and Stroke-Alzheimer's Disease and Related Disorders Association (NINCDS-ADRDA)¹⁵ (500 µL - 1 mL from each patient) was pooled for this study. The mean age in this group was 72.1 ± 6.6 years. The group consisted of 7 women and 3 men. The degree of memory impairment was assessed with mini-mental state examination in all AD cases resulting in the mean score of 14.6 ± 5.1. This indicates moderate-to-severe dementia. As a control, a pool of CSF was prepared from ten patients punctured due to other neurologic complaints without any signs of dementia. The mean age was 55.2±12.1 years, and there were 4 women and 6 men in the group. The pooled CSF was frozen and stored at -80 °C.

Brain homogenates were prepared from *postmortem* tissue according to the procedure described recently ¹⁰. Briefly, the *post mortem* tissue (~ 50mg) from the frontal lobe and cerebellum of brains of ten patients with neuropathologically confirmed AD^{16,17} and from four subjects who died due to diseases not related to dementia were prepared. Protein content of the samples was determined by the BCA assay ¹⁸ and adjusted to 3mg/mL.

### 2. Preparation of theProteinChip® Arrays and SELDI-TOF analysis

CSF and brain homogenates were subjected to antibody capture using a monoclonal antibody specific for the N-terminus of human Aβ peptides (6E10; Senetec, Maryland Heights, MO, USA), and the mass of each Aβ peptide species was determined using the ProteinChip Reader (Ciphergen Biosystems, Fremont, CA, USA)^{19, 20}. Affinity arrays were constructed by coupling the 6E10 antibody or bovine IgG control with a PS20 and/or PS10 ProteinChip Array. A 2-µL aliquot (0.5 mg/mL of 6E10 antibody in PBS) was incubated in a humidity chamber for 2 h at room temperature to allow covalent binding to the array. Unreacted sites were then blocked by incubating the entire array with 8 mL of 0.5 M ethanolamine in PBS (pH 8.0) on a rocking platform for 30 min at room temperature. The array was washed twice with 8 mL of PBS containing 0.5% (v/v) Triton X-100 for 10 min and once with 8 mL of PBS for 5 min. The array was then placed in a ProteinChip bioprocessor. CSF (450 µL per spot), and brain homogenates (50 µL per spot, 3mg/mL protein content) were loaded in each well of the bioprocessor. The samples were incubated overnight at 4°C with constant shaking. Each spot was then washed three times with PBS containing 0.5% Triton X-100 for 15 min and three times with PBS for 5 min. The ProteinChip array was then removed from the bioprocessor and washed twice with 1.0 mM HEPES, pH 7.0, for 1 min. After the array had dried, 2 x 0.5 µL of a 20 % saturated solution of α-cyano-4-hydroxycinnamic acid in 0.5 % (v/v) trifluoroacetic acid, 50 % (v/v) acetonitrile was applied to each spot. Mass analysis was performed by averaging a minimum of 100 shots on a PBSII ProteinChip Reader. Peptide standards (Aβ1-16, Aβ 1-40, and Aβ1-42) were used for external mass calibration. The imprecision of the molecular mass measurements was calculated as the mean of the ratios of difference between the obtained and the expected molecular mass of each peptide analyzed to the expected molecular mass of the peptide.

### 3. Aβ-SDS-PAGE/immunoblot

Aβ-SDS-PAGE/immunoblot was performed on the material from the same pools of CSF as that used for the SELDI-TOF analysis with the exception that the samples were exposed to one more freezing/thawing cycle before Aβ-SDS-PAGE/immunoblot.

For the separation of Aβ peptides, the urea version of the N,N'-bis-(2-hydroxylethyl)-glycine/bis-Tris/Tris/sulfate SDS-PAGE was used as described by Wiltfang et al. ²¹. Briefly, native CSF (10µL) in a sample buffer was applied to a gel slot. Gels were run at room temperature for 2 hours at a constant current of 12 mA/gel using the MiniProtean II electrophoresis unit (Bio-Rad). Next, semi-dry Western blotting was performed according to the protocol of Wiltfang et al. ²² using PVDF membranes. Immunostaining was performed with monoclonal amino-terminal-selective antibody, 1E8. After the washing step, the membranes were incubated with an anti-mouse biotinylated antibody, washed and horseradish peroxidase-coupled streptavidin was added for 1 h. After the final wash, the chemiluminescence was visualized with ECLPlus solution (Amersham Pharmacia, Sweden) according to the protocol of the manufacturer, using the CCD camera system (FluorSMax Multilmager, Bio-Rad).

### Results

### 1. The patterns of CSF Aβ peptides in AD and non-demented controls.

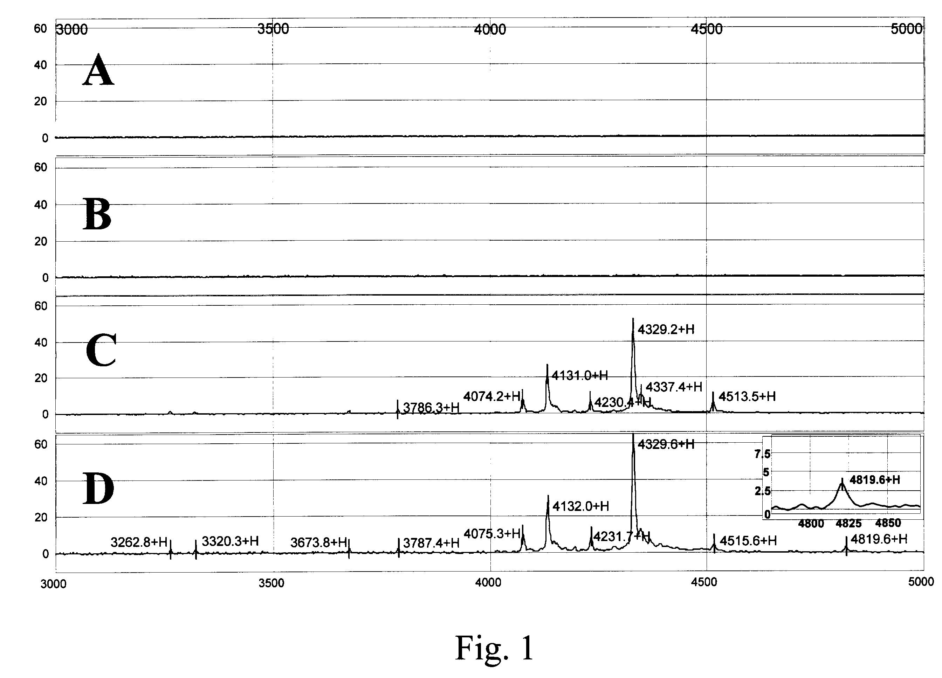

Incubation of either AD or control CSF with bovine IgG ProteinChip Array did not evoke any signal, as presented in Fig. 1 A and B, respectively, however, incubation of AD or control material with anti-Aβ antibody (6E10) coupled to ProteinChip Array evoked mass spectrometry signals. Therefore, it has to be assumed that these signals are related to Aβ peptides present in the investigated samples. The mean imprecision of molecular mass measurements was in the acceptable range, 431 x 10⁻⁶.

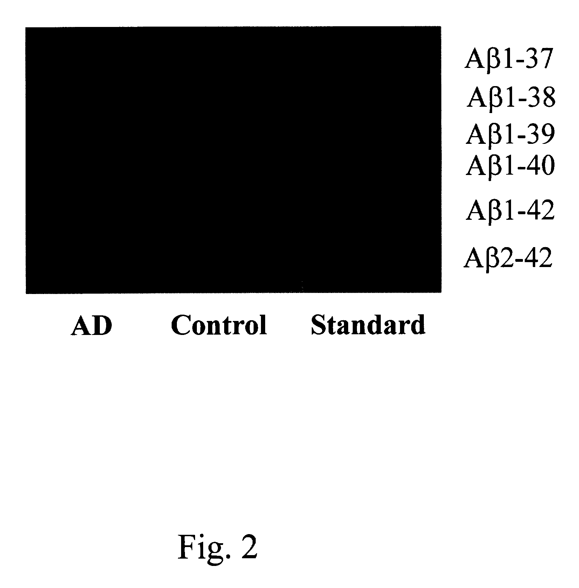

The difference in CSF Aβ peptide patterns between AD and non-demented controls is presented in Fig. 1 C and D and in Fig. 2 with regard to SELDI-TOF and Aβ-SDS-PAGE/immunoblot, respectively. The molecular masses of Aβ peptides are presented in Table 1. Application of SELDI-TOF for the analysis of Aβ peptides revealed ten distinct peaks on mass spectrometry that could be assigned as the following Aβ peptides based only on their molecular masses: 1-28, 5-34, 1-33, 1-34, 1-37, 1-38, 1-39, 1-40, 1-42, and 1-45 or 2-46. Interestingly, the last peptide with the observed molecular mass of 4819 6 Da, corresponding to either Aβ1-45 (expected molecular mass, 4811.62 Da) or Aβ2-46 (expected molecular mass, 4827.49 Da) was clearly visible only in the pooled CSF of AD, and it was not present in the pooled CSF of controls. The differences in evoked signals in the samples analyzed are presented in Table 2. In the CSF of AD a clear decrease of the signal coming from the peak corresponding to Aβ1-42 is present corresponding to the expected decrease of the concentration of this peptide in the CSF in AD. This is accompanied by an increased peak corresponding to Aβ1-38 and unchanged Aβ1-40, resulting in the decreased ratio of Aβ1-42/1-40. Similarly, Aβ-SDS-PAGE/ immunoblot revealed distinctly decreased density of the band corresponding to Aβ1-42 in CSF of AD. However, with the latter method only the bands corresponding to the peptides: Aβ1-37, Aβ1-38, Aβ1-39, Aβ1-40, and Aβ1-42 are visible. In this study, neither SELDI-TOF nor Aβ-SDS-PAGE/immunoblot has shown presence of Aβ2-42 peptide in CSF samples.

### 2. The patterns of Aβ peptides in brain homogenates of AD and controls.

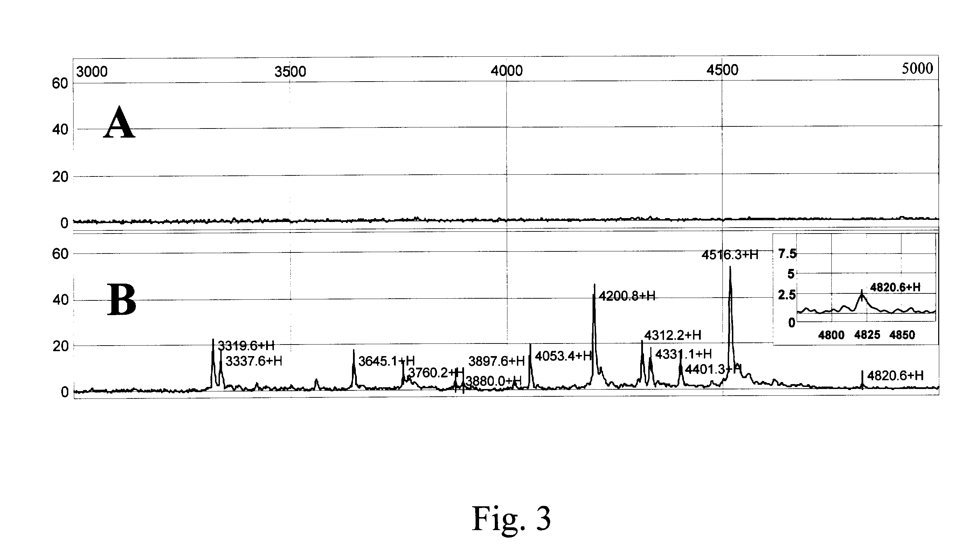

The patterns of Aβ peptides in human brain tissue from non-demented subjects and patients with AD are presented in Fig. 3 A and B, respectively. In the brain tissue of control patients, no Aβ peptides were observed, however, several peaks were present in the SELDI-TOF analysis of AD brains. The expected and obtained molecular masses of these peptides are presented in Table 1, and the differences in signals' intensities in the samples are presented in Table 2.

### Discussion

In this study, amyloid β peptides were analyzed in human CSF and brain tissue in Alzheimer's disease and in non-demented controls with SELDI-TOF method. The combination of SELDI-TOF mass spectrometry with the antibody specific for Aβ peptides results in evoking of SELDI-TOF signals related only to amyloid β peptides. For the first time with this technique, differences in the pattern of CSF Aβ peptides between AD and non-demented controls have been shown. Moreover, of a novel carboxyterminally elongated Aβ peptide corresponding to Aβ1-45 or Aβ2-46 according to its molecular mass has been found in pooled CSF and brain samples of AD, but neither in pooled CSF nor brain samples of controls.

Although the precise 'translation' of the SELDI-TOF MS signal intensity into a peptide concentration is not possible, it may be assumed that a clear decrease of the signal coming from Aβ1-42 in the CSF of AD compared to the control pool expectedly corresponds to the decreased concentration of this peptide. The decreased concentration of Aβ1-42 in CSF of patients with Alzheimer's disease has been reported by many investigators, and the finding in this study is consistent with previous reports^{8, 10, 11, 12}. Recently, the decreased concentration of Aβ1-42 at unchanged Aβ1-40 and total Aβ peptides have been reported. Moreover, the ratio of Aβ1-38 to the total Aβ peptides in CSF of AD was increased ⁸. Here, the mass spectrometry analysis suggests the presence of another, so far not reported Aβ peptide corresponding to Aβ1-45 or 2-46 according to its molecular mass. The mechanisms of these changes in Aβ peptides concentrations in AD are not clear so far. A previous explanation postulating a simple accumulation of Aβ1-42 in plaques needs to be verified, since decreased concentrations of this peptide have recently been reported in CSF of patients with Creutzfeldt-Jakob disease who did not show any β-amyloid plaques ²³. As an explanation, it may be hypothesized that both neurodegenerative diseases may share a similar chaperon with a high avidity binding of misfolded molecules that decreases their detectable fraction due to epitope masking. Interestingly, a tendency of proteins to become toxic when they misfold and aggregate has recently been reported for other molecules ²⁴. Alternatively, the proteolytic generation and catabolism of discrete Aβ peptide species, like Aβ1-42 and Aβ1-45 can be affected differentially. This hypothesized shift would favor release of other species of Aβ peptides from APP at the expense of Aβ1-42, disturbing the balance among Aβ peptides. Such shifts in activities of secretases under pathologic conditions has been observed in the cell culture model ⁸.

Several Aβ species have been found in the pooled brain homogenates of the AD subjects but not in controls. This is in agreement with the previous report showing that the concentration of the most abundant Aβ peptide, i.e. Aβ1-42 is increased forty-fold in AD compared to controls ¹⁰. interestingly, some of the Aβ peptides observed by us in the brain have recently been reported to exist in the skeletal muscles of AD patients²⁵. In spite of a free diffusion of molecules between the brain parenchyma and the ventricular CSF, only a subset of Aβ species observed in the brain were also present in the CSF of AD. This might be explained by a rapid metabolism of these peptides within the brain tissue before entering the CSF. Moreover, the composition of lumbar CSF differs significantly from the CSF produced within the brain ventricles due to out-flux of molecules during the bulk flow of CSF within the subarachnoidal space ²⁶. Like in Kuo et al. ²⁵, *postmortem* tissue was also used here. Therefore, unpredictable secondary proteolytic processes may generate additional Aβ peptide fragments observed *postmortem* in the brain tissue of AD patients.

Comparison of SELDI-TOF and Aβ-SDS-PAGE/immunoblot shows that the former method is superior with regard to resolving a complex pattern of Aβ peptides in biological material. Further, the precise molecular mass information provided by SELDI-TOF supports the identification of the unknown peptide species. However, the pooled CSF which was used for Aβ-SDS-PAGE/immunoblot was exposed to one freeze/thaw cycle more compared to the material used for SELDI-TOF. Therefore, it cannot be excluded that the absence of the band corresponding to the Aβ1-45 in Aβ-SDS-PAGE/immunoblot is related to cold precipitation processes. In this study, neither SELDI-TOF nor Aβ-SDS-PAGE/immunoblot has shown the presence of Aβ2-42 peptide in AD samples. This may be explained by the fact that only approximately 30% of AD patients posses Aβ2-42 in the CSF ¹⁰.

Concluding, it may be suggested that SELDI-TOF will be a promising tool for the analysis of Aβ peptide expression patterns in patients with Alzheimer's disease. This technology will allow placing a combination of different monoclonal antibodies on the same chip to reveal disease-specific expression patterns in minute amounts of biological material.

### Second Study

### Overview

In the second study, cerebrospinal fluid (CSF) from patients with AD (n=10) and non-demented controls (CON, n=9) was studied by the method of surface enhanced laser desorption/ionization-time of flight mass spectrometry (SELDI-TOF MS). Three molecular mass signals were observed corresponding to three novel previously not described amyloid beta (Aβ) peptides with molecular masses 4525.1 Da, 4846.8 Da and 7755.8 Da (Aβ(4525.1), Aβ(4846.8) and Aβ(7758.8+2H), respectively). The signal-to-noise ratios of Aβ(4525.1) and Aβ(7758.8+2H) were significantly decreased in AD (Aβ(4525.1): median 2.2 and 4.3 in AD and controls, respectively, p<0.0005; Aβ(7758.8+2H): median 1.0 and 14.0 in AD and controls, respectively, p<0.000023), whereas the signal-to-noise ratio of Aβ(4846.8) was significantly increased in AD (median 3.6 and 2.5 in AD and controls, respectively, p<0.044). The molecular masses of Aβ(4525.1) and Aβ(4846.8) may correspond to Aβ3-44 and Aβ3-47, respectively, and the molecular mass of Aβ(7758.8+2H) is in accordance with the molecular mass of dimeric Aβ, however, the precise amino acid sequence of these peptides is now under evaluation. To confirm that SELDI-TOF MS may be used as a quantitative method, signal-to-noise intensities of two generally accepted biomarkers, Aβ1-42 and Aβ1-40 which, expectedly, turned out to be significantly decreased (p<0.008) and unaltered in AD (p=0.36) were evaluated, respectively. Moreover, direct comparison of Aβ1-42 results obtained with SELDI-TOF MS and ELISA showed a moderate but highly significant correlation between results of both measurements (R=0.66, p<0.002). Significant differences in the signal-to-noise ratios of other Aβ peptides known to be present in the CSF (i.e. Aβ1-37/38/39) have not been observed. Concluding, there are two novel previously not published amyloid beta peptides, which may play important role in the diagnosis of Alzheimer's disease.

### Introduction

In surface enhanced laser desorption/ionization time-of-flight mass spectrometry (SELDI-TOF MS), a combination of immune absorption of biological molecules (e.g. peptides) with extremely precise analysis of their molecular mass allows characterization of novel biomarkers in biological material (Li et al., 2002; Qu et al., 2002). In this study SELDI-TOF MS was applied to analyze pattern of Aβ peptides in CSF of patients with clinically established and neurochemically supported diagnosis of AD. Two novel previously not described peptides with molecular mass corresponding to Aβ-44 and Aβ3-47 were found in CSF, and signal-to-noise ratio of these peptides were significantly altered in AD compared to non demented controls. Moreover, mass spectrometry signal-to-noise ratio of a known AD biomarker, Aβ1-42 was expectedly found decreased in AD.

### Results

Figure 4 shows fragments of representative SELDI-TOF spectra of two patients out of 19 analyzed in this study. Incubation of CSF from a control subject on an array with unspecific bovine IgG did not evoke any significant signal, as presented in Fig. 4a, 4b and 4c. However, incubation of CSF of this same subject on an array with monoclonal antibody specific for Aβ peptide (6E10) resulted in clear mass spectrometry signals as presented in Figure 4d, 4e and 4f. Similarly, incubation of CSF from a patient with AD with the array pre-coated with 6E10 evoked mass spectrometry signals as presented in Fig. 4g, 4h and 4i. Four novel previously not reported mass spectrometry peaks were observed in this study corresponding to peptides with molecular masses of 4525.1 Da, 4846.8Da, 7755.8 Da and 3882.2 Da. However, it cannot be excluded that the signal at 3882.2 Da represents a doubly charged peptide with molecular mass of 7755.8 Da and, therefore, currently both these signals must be treated as resulting from one Aβ peptide.

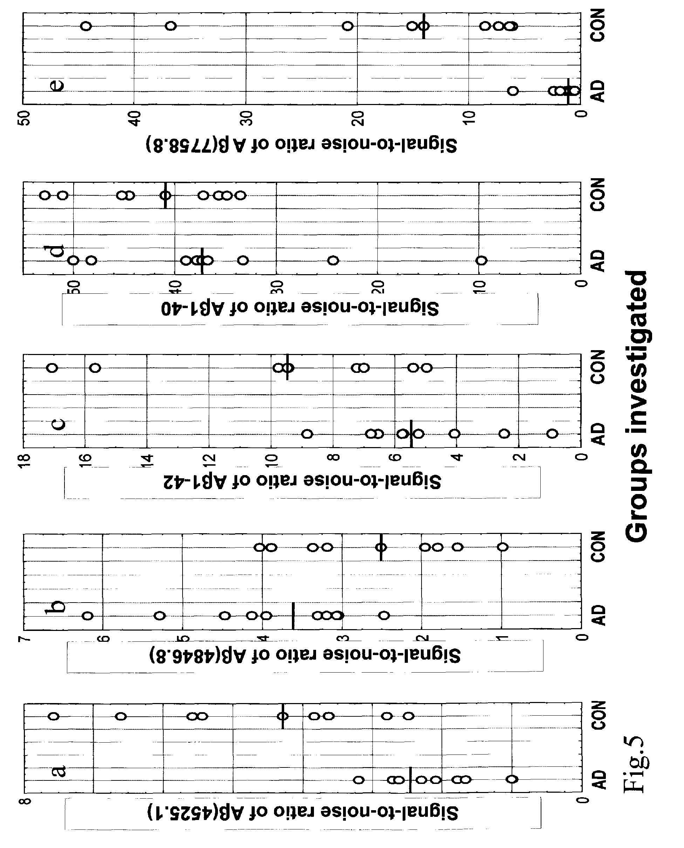

Figure 5 presents SELDI-TOF MS signal-to-noise ratios of Aβ peptide with molecular mass 4525.1 Da (Aβ(4525.1)) which was significantly decreased (Fig. 5a), and signal-to-noise ratio of Aβ peptide with molecular mass 4846.8 Da (Aβ(4846.8)) which was significantly increased (Figure 5b) in AD compared to controls. Signal-to-noise ratios of two peptides commonly used as AD biomarkers, namely Aβ1-42 (Figure 5c) and Aβ1-40 (Figure 5d) were decreased and unaltered, respectively, in patients with AD. The signal-to-noise ratio of Aβ peptide with molecular mass 7755.8 (Aβ(7755.8+2H)) was significantly decreased in AD (Figure 5e), and actually no overlap of the cases was observed between both groups. Statistical evaluation of the data is presented in Table 3. The observation of decreased signal-to-noise ratio of Aβ1-42 in CSF in AD is consonant with previous findings of decreased concentration of this biomarker reported by our group (Wiltfang et al., 2001; Wiltfang et al., 2002) as well as other investigators (Andreasen et al., 2001; Hulstaert et al., 1999). To further explore a potential of quantitative measurements by SELDI-TOF MS, a direct comparison of Aβ1-42 results obtained with SELDI-TOF MS and ELISA has been performed to show a moderate but highly significant correlation between concentration (as measured with ELISA) and signal-to-noise ratio of this biomarker (Spearman rank order correlation R=0.66, p<0.002). Therefore, it may be considered that the altered signal-to-noise ratios of Aβ peptides originate from altered CSF concentrations also in case of other Aβ peptides. This might have important consequences for neurochemical diagnosis of AD, since the concentration of these novel biomarkers cannot be measured with any other method available so far. No significant alterations in the signal-to-noise ratios of other Aβ peptides known to occur in the CSF (i.e. Aβ1-37/38/39) were observed between the groups studied (Table 3).

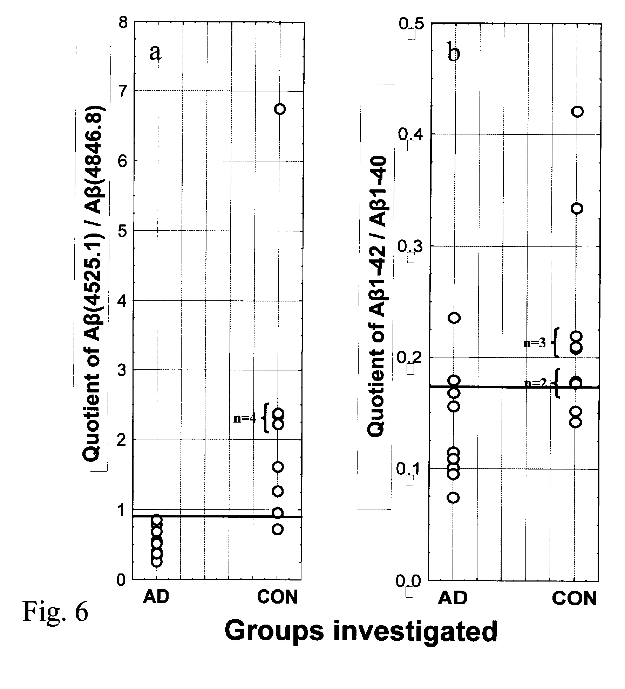

To compensate possible influences of deviations of chemical compounds used in the method as well as physical properties of the SELDI-TOF MS reader (e.g. laser energy deviations, detector sensitivity imprecision etc.), the quotients of the signal-to-noise ratios of the peptides have also been applied to further improve separation of patients. This resulted in a better resolution of both groups studied, which is not surprising since such an approach has already been successfully applied in combination with other analytical methods (e.g. ELISA, SDS-PAGE-immunoblot) used in neurochemical diagnosis of dementias (Shoji et al., 1998; Wiltfang et al., 2002) As presented in Figure 6, with the cut off value 0.95 the quotient of signal-to-noise ratios Aβ (4525.1) / Aβ(4846.8) (Figure 6a) classified properly 18/19 (94.7%) patients whereas with the cut off value 0.175 the quotient Aβ1-42/Aβ1-40 (Figure 6b) classified correctly 15/19 (79%) of patients.

Peptides investigated in this study are clearly specifically immunoreactive with monoclonal antibody against Aβ peptides but not with unspecific IgG. According to their molecular masses, one might speculate on their amino acid sequence, which could correspond to Aβ3-44 (expected molecular mass, 4528.2 Da; difference between the measured and the expected molecular masses < 0.07%) and Aβ3-47 (expected molecular mass, 4853.6 Da; difference between the measured and the expected molecular masses < 0.14%). In such a case, one might hypothesize that in AD a cutting position of secretases (in this case, γ-secretase) is shifted, and the longer Aβ peptides are released on the expense of the shorter species. Interestingly, in AD a dramatic decrease of Aβ peptide with molecular mass exceeding molecular masses of all monomeric Aβ peptides reported by any publication so far was found. Therefore, it may be hypothesized that this mass spectrometry signal represents a dimeric form of Aβ peptides, as such forms have been reported to be present in the CSF (Vigo-Pelfrey et al., 1993). The decrease of the signal-to-noise ratio of this peptide, representing the best separating parameter reported so far, may result from decreased synthesis, increased turnover or dissociation of dimeric form into monomeric form(s). However, given the number of possible post-translational modifications of Aβ peptides, such a speculation must be considered extremely carefully, and the eventual amino acid composition may be only obtained in further studies.

Expectedly, in our study the ELISA concentrations of Aβ42 in CSF were significantly lower in AD (median, 434, range, 259-537 pg/mL) compared to the controls (median, 889, range, 700-1902 pg/mL, p<0.00003). As an explanation of decreased concentration of Aβ1-42 in CSF of AD patients, a hypothesis was suggested that this decrease resulted from accumulation of Aβ1-42 in senile plaques. However, our recently published results that Aβ1-42 is significantly decreased also in the CSF of patients with Creutzfeldt-Jakob disease who did not have plaques suggest that a simple accumulation of Aβ peptides in plaques is not sufficient explanation, and other mechanisms, like e.g. changes in chaperons' functions, must also be considered in both neurodegenerative disorders (Otto et al., 2000).

Concluding, two novel previously not reported amyloid β peptides with molecular mass corresponding to Aβ3-44 and Aβ3-47 in human CSF are described, and it is hypothesized that these novel peptides may play an important role in neurochemical diagnosis of Alzheimer's disease as well as for understanding its pathophysiology.

### Materials and methods

### 1. Patients and CSF;

The study was approved by the ethics committee of the University of Goettingen. All patients gave their informed consent. CSF was obtained from all patients by a lumbar puncture as a part of a routine diagnostic procedure. CSF was aliquoted, immediately frozen and stored at -80°C until assay.

The group of Alzheimer's disease (AD, n=10) consisted of patients diagnosed according to the criteria of ICD-10, and National Institute of Neurological and Communicative Disorders and Stroke-Alzheimer's Disease and Related Disorders Association (NINCDS-ADRDA) (McKhann et al., 1984). The degree of mental impairment was assessed with the Mini Mental State Examination (MMSE) (Folstein et al., 1975). There were 8 women and 2 men in this group. Median age of AD patients was 68 years (range, 51-89).

The control group (CON, n=9) comprised patients with neurologic or psychiatric diseases but without memory complaints. There were 5 women and 4 men in this group. Median age of control group was 59 years (range, 25-81). The patients of this group were slightly younger, however, the age did not differ significantly (p=0.08). All subjects underwent extensive dementia investigation including careful clinical examination, routine blood, urine and CSF tests, magnetic resonance imaging or computed tomography and neuropsychological tests when applicable. Routine CSF / serum analysis was performed according to Reiber (Reiber and Peter, 2001) including assessment of blood - CSF barrier function and symptoms indicating neuroinflammatory disorders.

### 2. Preparation of the Protein Chip® Arrays, SELDI-TOF analysis and evaluation of spectra

2µL of Aβ N-terminus specific monoclonal antibody (6E10; Senetec, Maryland Heights, MO, USA) or unspecific bovine IgG was incubated in a humidity chamber for 2 h at room temperature to allow covalent binding to the PS20 ProteinChip® array (Ciphergen Biosystems, Fremont, CA, USA). Unreacted sites were then blocked by incubating the entire array with 8 mL of 0.5 M ethanolamine in PBS (pH 8.0) on a rocking platform for 30 min at room temperature. The array was washed twice with 8 mL of PBS containing 0.5% (v/v) Triton X-100 for 10 min and once with 8 mL of PBS for 5 min. The array was then placed in a ProteinChip bioprocessor and 50 µL of CSF or peptide standards for external mass calibration (Aβ1-16, Aβ 1-40, and Aβ1-42) were applied per spot. The samples were incubated overnight at 4°C with constant shaking. Each spot was then washed three times with PBS containing 0.5% Triton X-100 for 15 min and three times with PBS for 5 min. The ProteinChip array was then removed from the bioprocessor and washed several times with 1.0 mM HEPES, pH 7.0. After the array had dried, 1 µL of a 20 % saturated solution of α-cyano-4-hydroxycinnamic acid in 0.5 % (v/v) trifluoroacetic acid, 50 % (v/v) acetonitrile was applied to each spot. Mass analysis was performed by averaging a minimum of 100 shots on a PBSII ProteinChip Reader (Ciphergen Biosystems). All samples in this study were measured with the reader set at this same laser energy (210), the detector sensitivity (7) and optimization range (1000-7500 Da). The spectra were evaluated with Ciphergen ProteinChip Software Biomarker 3.0.1, and for statistical analysis peaks' signal-to-noise ratios were considered. The sequence of Aβ peptides best fitting to the molecular mass obtained with SELDI-TOF MS was evaluated with internal software (AβCalculator).

### 3. Aβ42 ELISA;

Aβ42 was measured in all cases with a commercially available ELISA kit (Abeta GmbH, Heidelberg, Germany). Briefly, 50 µL of undiluted CSF from patients or standards included in the kit were applied to microtiter plates pre-coated with antibody specifically recognizing N-terminus of Aβ peptides, WO-2 (Ida et al., 1996; Jensen et al., 2000). Following an overnight incubation at 4°C and washing steps, detection antibody indirectly linked to an enzyme was applied. This antibody, G2-13 (isotype, IgG1, κ), has its epitope localized at the carboxyl terminus of the Aβ42 peptide, and thus, specifically recognizes this peptide. After incubation and washing, chromogene was added to incubate 15-20 minutes, and the reaction was stopped with sulfuric acid. The color reaction was measured with an automatic ELISA reader (Benchmark, Bio Rad, USA) with the wavelength set at 450 nm and a reference wavelength of 655 nm. The software (Microplate Manager, Bio Rad, USA) was used to create standard curves and to calculate the concentration of the samples.

### 4. Statistical analysis;

Results are presented as medians and ranges. Comparisons between groups were performed with Mann-Whitney U test using Statistica 6.0 (StatSoft). A p value less than 0.05 was considered significant. Molecular masses of peptides measured with SELDI-TOF MS are presented as means of masses obtained in all spectra analyzed. The imprecision of the measurements of molecular masses was calculated as the mean of the differences between the obtained and the expected molecular mass of each peptide divided by the expected molecular mass of the peptide.

### Summary of both Studies

Amyloid β (Aβ) peptides have been investigated in human body fluid (cerebrospinal fluid, CSF) and *post-mortem* brain tissue of patients with Alzheimer's disease (AD) and non-demented controls (CON) with surface enhanced laser desorption/ionization time-of-flight mass spectrometry (SELDI-TOF MS) in two independent experiments (first and second study).

The molecular masses and the signal intensities of Aβ peptides have been measured with ProteinChip Reader (Ciphergen Biosystems, Fremont, CA, USA).

In the pooled CSF and in the brain tissue of AD patients (n=10 for each group), Aβ peptide has been identified with the molecular mass 4819.6 (range, 4802-4836) existing neither in pooled CSF (n=10) nor brain tissue (n=10) of controls. According to the expected molecular mass, this peptide corresponds most probably to Aβ2-46, however, Aβ1-45 cannot be excluded.

Additionally, alterations of four Aβ peptides' signals have been identified in individual CSF samples from AD (n=10) and non demented (n=9) subjects (E2) with molecular masses 4525.1 (range, 4520-4545, decreased in AD, p=0.0005), 4846.8 (range, 4837-4871, increased in AD, p=0.044), 3882.2 (range, 3870-3895, decreased in AD, p=0.000023), and 7755.8 (range, 7720-7790, decreased in AD, p =0.00009). It is possible, however, that the peak with molecular mass of 3882.2 represents a signal of a doubly charged Aβ peptide with molecular mass of 7755.8 (Aβ7755.8 + 2H). Probably, the signals with molecular masses of 4525.1 and 4846.8 represent Aβ3-44 and Aβ3-47, respectively. At Aβ(7755.8 + 2H) cut off value of 6.1 (range 4 - 7.8, cut off ± 30%), the full separation of the patients of both groups has been observed (100% sensitivity and 100% specificity). The quotient of signal-to-noise ratios of Aβ(4525.1) to Aβ(45846.8) (R Aβ(4525.1 )/Aβ(45846.8)) allowed a better discrimination of patients of both groups (AD and CON) compared to any of Aβ peptides analyzed alone (E2). At the cut off value of 0.95 (range, 0.665-1.235, cut off ± 30%), the patients of both groups have been discriminated with specificity of 88.9% and sensitivity of 100% (p=0.0002).

The application of the quotient of signal-to-noise ratios of Aβ peptides eliminates possible changes of physical and chemical properties of individual measurements, since these individual changes influence all Aβ peptides similarly. Therefore, it is expected that this approach will allow quantitative Aβ analysis. Since it is known that in AD the concentrations of Aβ1-40 and Aβ1-42 are unaltered and decreased, respectively, the SELDI-TOF MS signal-to-noise ratios of these peptides have been analyzed and, expectedly, have been found in AD unaltered and decreased (p=0.008), respectively. At the cut off value of 0.175 (range, 0.123 - 0.3; cut off ± 30%), the quotient of signal-to-noise ratios of Aβ1-42/Aβ1-40 discriminates the patients of both groups with specificity of 77.8% and sensitivity of 80% (p=0.014).

**Table 1.**

| Aβ peptides in human CSF and brain tissue. | | |
|---|---|---|
| Observed Mᵣ^{a} | Expected Mᵣ | Aβ sequence |
| CSF: | | |
| 3262.8 | 3262.5 | 1-28 |
| 3320.3 | 3324.75 | 5-34 |
| 3673.8 | 3674.0 | 1-33 |
| 3787.4 | 3787.18 | 1-34 |
| 4075.3 | 4074.5 | 1-37 |
| 4132.0 | 4131.6 | 1-38 |
| 4231.7 | 4230.9 | 1-39 |
| 4329.6 | 4329.9 | 1-40 |
| 4515.6 | 4514.1 | 1-42 |
| 4819.6 | 4811.62 or | 2-46 or |
| | 4827.49 | 1-45 |

| Brain: | | |
|---|---|---|
| 3319.6 | 3324.75 | 5-34 |
| 3645.1 | 3643.1 | 8-42 |
| 3760.2 | 3758.18 | 7-42 |
| 4053.4 | 4051.67 | 5-42 |
| 4200.8 | 4198.84 | 4-42 |
| 4312.2 | 4310.0 | 3p-42^{b} |
| 4331.1 | 4329.9 | 1-40 |
| 4401.3 | 4399.02 | 2-42 |
| 4516.3 | 4514.1 | 1-42 |
| 4820.6 | 4811.62 or 4827.49 | 2-46 or 1-45 |

| | | |
|---|---|---|
| ^{a} Mᵣ, molecular mass (Da) | | |
| ^{b} Aβ3p-42, the pyroglutamate derivative of Aβ3-42 | | |

**Table 2.**

| Comparison of SELDI-TOF MS signal intensities of Aβ peptides in human CSF and brain tissue in AD and control pools | | | | |
|---|---|---|---|---|
| | Signal intensity | | | |
| Aβ peptide | CSF | | Brain tissue | |
| | AD | Control | AD | Control |
| 1-28 | 234 | ND | ND | ND |
| 5-34 | 219 | ND | 17.6 | ND |
| 1-33 | 2.3 | ND | ND | ND |
| 1-34 | 2.86 | 238 | ND | ND |
| 1-37 | 10.03 | 7.95 | ND | ND |
| 1-38 | 26.42 | 21.6 | ND | ND |
| 1-39 | 8.80 | 6.74 | ND | ND |
| 1-40 | 59.9 | 47.33 | 13.35 | ND |
| 1-42 | 4.56 | 6.43 | 48.4 | ND |
| 2-46 or 1-45 | 3.39 | ND | 2.28 | ND |
| 8-42 | ND ^{a} | ND | 1234 | ND |
| 7-42 | ND | ND | 7.48 | ND |
| 5-42 | ND | ND | 14.65 | ND |
| 4-42 | ND | ND | 40.7 | ND |
| 3p-42 ^{b} | ND | ND | 16.07 | ND |
| 2-42 | ND | ND | 11.9 | ND |

| | | | | |
|---|---|---|---|---|
| ^{a} ND, not detected | | | | |
| ^{b} Aβ3p-42, the pyroglutamate derivative of Aβ3-42 | | | | |

**Table 3.**

| **Molecular masses and signal-to-noise ratios of the amyloid β peptides.** | | | | | | |
|---|---|---|---|---|---|---|
| Peptide | Mean mass measured | Mass expected | Mass imprecision x 10^{-6 A)} | Signal-to-noise ratio, median (range) | | |
| | (Da) | (Da) | | Control | AD | p ^{B}) |
| Aβ3-44 | 4525.1 | 4528.2 | -684 | 4.3 | 2.2 | 0.0005 |
| | | | | (2.5-7.6) | (1.0-3.2) | |
| Aβ3-47 | 4846.8 | 4853.6 | -1393 | 2.5 | 3.6 | 0.044 |
| | | | | (1.0-4.0) | (2.5-6.2) | |
| Aβ1-42 | 4512.0 | 4514.1 | -475 | 9.4 | 5.5 | 0.008 |
| | | | | (5.0-17.1) | (0.9-8.8) | |
| Aβx-x^{C)} | 7755.8 | -- | -- | 14.0 | 1.0 | 0.000023 |
| | | | | (6.13 - 44.4) | (0.6 - 6.08) | |
| Aβ1-37 | 4071 8 | 4074.5 | -667 | 11.1 | 10.7 | 0.36 |
| | | | | (7.6 - 23.3) | (4.8 - 13.4) | |
| Aβ1-38 | 4129.0 | 41316 | -627 | 26.8 | 26.5 | 0.60 |
| | | | | (16.8-40.3) | (5.0 - 29.3) | |
| Aβ-39 | 4228.4 | 4230.9 | -585 | 10.3 | 9.3 | 0.50 |
| | | | | (4.5 - 16.5) | (1.7 - 12.6) | |
| Aβ1-40 | 4327.2 | 4329.9 | -614 | 41.0 | 37.4 | 0.36 |
| | | | | (33.5 - 52.9) | (9.8 - 50.0) | |

| | | | | | | |
|---|---|---|---|---|---|---|
| ^{A}) Mass imprecision = (mean mass measured - mass expected) / mass expected; | | | | | | |
| ^{B}) Mann-Whitney U test; | | | | | | |
| ^{C}) Precise amino acid sequence of this Aβ peptide is currently under analysis. | | | | | | |

### References

### References for the first Study

1. see reference (3) for the second study.
2. see reference (19) for the second study.
3. see reference (1) for the second study.
4. see reference (22) for the second study.
5. see reference (18) for the second study.
6. see reference (11) for the second study.
7. see reference (6) for the second study.
8. see reference (23) for the second study.
9. Riemenschneider M, Schmolke M, Lautenschlager N, Guder WG, Vanderstichele H, Vanmechelen E, Kurz A. Neurosci Lett 2000; **284**: 85-8.
10. see reference (21 ) for the second study.
11. see reference (2) for the second study.
12. Galasko D, Chang L, Motter R, Clark CM, Kaye J, Knopman D, Thomas R, Kholodenko D, Schenk D, Lieberburg I, Miller B, Green R, Basherad R, Kertiles L, Boss MA, Seubert P. Arch Neurol 1998; **55**: 937-45.
13. see reference (13) for the second study.
14. see reference (5) for the second study.
15. see reference (22) for the second study.
16. Braak H, Braak E. Acta Neuropathol (Berl) 1991; **82**: 239-59.
17. Mirra SS, Heyman A, McKeel D, Sumi SM, Crain BJ, Brownlee LM, Vogel FS, Hughes JP, van Belle G, Berg L. Neurology 1991; **41**: 479-86.
18. Smith PK, Krohn RI, Hermanson GT, Mallia AK, Gartner FH, Provenzano MD, Fujimoto EK, Goeke NM, Olson BJ, Klenk DC. Anal Biochem 1985; **150**: 76-85.
19. Austen BM, Frears ER, Davies H. J Pept Sci 2000; **6**: 459-69.
20. Davies H, Lomas L, Austen B. Biotechniques 1999; **27**: 1258-61.
21. Wiltfang J, Arold N, Neuhoff V. Electrophoresis 1991; **12**: 352-66.
22. Wiltfang J, Smirnov A, Schnierstein B, Kelemen G, Matthies U, Klafki HW, Staufenbiel M, Hüther G, Rüther E, Kornhuber J. Electrophoresis 1997; **18**: 527-32.
23. see reference (14) for the second study.
24. Bucciantini M, Giannoni E, Chiti F, Baroni F, Formigli L, Zurdo J, Taddei N, Ramponi G, Dobson CM, Stefani M. Nature 2002; **416**: 507-11.
25. see reference (9) for the second study.
26. Reiber H. Clin Chim Acta 2001; **310**: 173-86.

### References for the second Study

(1) (**1998**) Consensus report of the Working Group on: "Molecular and Biochemical Markers of Alzheimer's Disease". The Ronald and Nancy Reagan Research Institute of the Alzheimer's Association and the National Institute on Aging Working Group. *Neurobiol Aging* 19, 109-16.
(2) Andreasen N., Minthon L., Davidsson P., Vanmechelen E., Vanderstichele H., Winblad B. & Blennow K. (**2001**) Evaluation of CSF-tau and CSF-Aβ42 as diagnostic markers for Alzheimer disease in clinical practice. *Arch Neurol* 58, 373-9.
(3) Blennow K., Vanmechelen E. & Hampel H. **(2001)** CSF total tau, Aβ42 and phosphorylated tau protein as biomarkers for Alzheimer's disease. *Mol Neurobiol* 24, 87-97.
(4) Folstein M.F., Folstein S.E. & McHugh P.R. **(1975)** "Mini-mental state". A practical method for grading the cognitive state of patients for the clinician. *J Psychiatr Res* 12, 189-98.
(5) Frears E.R., Stephens D.J., Walters C.E., Davies H. & Austen B.M. (**1999**) The role of cholesterol in the biosynthesis of beta-amyloid. *Neuroreport* 10, 1699-705.
(6) Hulstaert F., Blennow K., Ivanoiu A., Schoonderwaldt H.C., Riemenschneider M., De Deyn P.P., Bancher C., Cras P., Wiltfang J., Mehta P.D., Iqbal K., Pottel H., Vanmechelen E. & Vanderstichele H. **(1999)** Improved discrimination of AD patients using beta-amyloid(1-42) and tau levels in CSF. *Neurology* 52, 1555-62.
(7) Ida N., Hartmann T., Pantel J., Schröder J., Zerfass R., FörstI H., Sandbrink R., Masters C.L. & Beyreuther K. (**1996**) Analysis of heterogeneous βA4 peptides in human cerebrospinal fluid and blood by a newly developed sensitive Western blot assay. *J Biol Chem* 271, 22908-14.
(8) Jensen M., Hartmann T., Engvall B., Wang R., Uljon S.N., Sennvik K., Näslund J., Muehlhauser F., Nordstedt C., Beyreuther K. & Lannfelt L. (2000) Quantification of Alzheimer amyloid β peptides ending at residues 40 and 42 by novel ELISA systems. *Mol Med* 6, 291-302.
(9) Kuo Y.M., Kokjohn T.A., Watson M.D., Woods A.S., Cotter R.J., Sue L.I., Kalback W.M., Emmerling M.R., Beach T.G & Roher A.E. (**2000**) Elevated abeta42 in skeletal muscle of Alzheimer disease patients suggests peripheral alterations of AbetaPP metabolism. *Am J Pathol* 156, 797-805.
(10) Li J., Zhang Z., Rosenzweig J., Wang Y.Y. & Chan D.W. (**2002**) Proteomics and bioinformatics approaches for identification of serum biomarkers to detect breast cancer. *Clin Chem* 48, 1296-304.
(11) Lichtenthaler S.F., Wang R., Grimm H., Uljon S.N., Masters C.L. & Beyreuther K. **(1999)** Mechanism of the cleavage specificity of Alzheimer's disease gamma-secretase identified by phenylalanine-scanning mutagenesis of the transmembrane domain of the amyloid precursor protein. *Proc Natl Acad Sci U S A* 96, 3053-8.
(12) McKhann G., Drachman D., Folstein M., Katzman R., Price D. & Stadlan E.M. (**1984**) Clinical diagnosis of Alzheimer's disease: report of the NINCDS-ADRDA Work Group under the auspices of Department of Health and Human Services Task Force on Alzheimer's Disease. *Neurology* 34, 939-44.
(13) Merchant M. & Weinberger S.R. (2000) Recent advancements in surface-enhanced laser desorption/ionization-time of flight-mass spectrometry. *Electrophoresis* 21, 1164-77.
(14) Otto M., Esselmann H., Schulz-Shaeffer W., Neumann M , Schroter A., Ratzka P., Cepek L., Zerr I., Steinacker P., Wind) O., Kornhuber J., Kretzschmar H.A., Poser S. & Wiltfang J. **(2000)** Decreased beta-amyloid1-42 in cerebrospinal fluid of patients with Creutzfeldt-Jakob disease. *Neurology* 54, 1099-102.
(15) Qu Y., Adam B.L., Yasui Y., Ward M.D., Cazares L.H., Schellhammer P.F., Feng Z., Semmes O.J. & Wright G.L., Jr. (**2002**) Boosted decision tree analysis of surface-enhanced laser desorption/ionization mass spectral serum profiles discriminates prostate cancer from noncancer patients. *Clin Chem* 48, 1835-43.
(16) Reiber H. & Peter J.B. **(2001)** Cerebrospinal fluid analysis: disease related data patterns and evaluation programs *J Neurol Sci* 184, 101-122.
(17) Shoji M., Matsubara E., Kanai M., Watanabe M., Nakamura T., Tomidokoro Y., Shizuka M., Wakabayashi K., Igeta Y., Ikeda Y., Mizushima K., Amari M., Ishiguro K., Kawarabayashi T., Harigaya Y., Okamoto K. & Hirai S. **(1998)** Combination assay of CSF tau, Aβ1-40 and Aβ1-42(43) as a biochemical marker of Alzheimer's disease. *J Neurol Sci* 158, 134-40.
(18) Tischer E. & Cordell B **(1996)** Beta-amyloid precursor protein. Location of transmembrane domain and specificity of gamma-secretase cleavage. *J Biol Chem* 271, 21914-9.
(19) Vanmechelen E., Vanderstichele H., Hulstaert F., Andreasen N., Minthon L, Winblad B., Davidsson P. & Blennow K. (**2001**) Cerebrospinal fluid τ and β-amyloid₍₁₋₄₂₎ in dementia disorders. *Mech Ageing Dev* 122, 2005-11.
(20) Vigo-Pelfrey C., Lee D., Keim P., Lieberburg I. & Schenk D.B. (**1993**) Characterization of beta-amyloid peptide from human cerebrospinal fluid. *J Neurochem* 61, 1965-8.
(21) Wiltfang J., Esselmann H., Cupers P., Neumann M., Kretzschmar H., Beyermann M., Schleuder D., Jahn H., Rüther E., Kornhuber J., Annaert W., De Strooper B. & Saftig P. **(2001)** Elevation of β-amyloid peptide 2-42 in sporadic and familial Alzheimer's disease and its generation in PS1 knockout cells. *J Biol Chem* 276, 42645-57.
(22) Wiltfang J., Esselmann H., Maler J.M., Bleich S., Hüther G. & Kornhuber J. **(2001)** Molecular biology of Alzheimer's dementia and its clinical relevance to early diagnosis and new therapeutic strategies. *Gerontology* 47, 65-71.
(23) Wiltfang J., Esselmann H., Bibl M., Smirnov A., Otto M., Paul S., Schmidt B., Klafki H.-W., Maler M., Dyrks T., Bienert M., Beyermann M., Rüther E. & Kornhuber J. (**2002**) Highly conserved and disease-specific patterns of carboxyterminally truncated A β peptides 1-37/38/39 in addition to 1-40/42 in Alzheimer's disease and in patients with chronic neuroinflammation. *J Neurochem* 81, 481-96.

## Claims

1. A method of diagnosis of Alzheimer's disease comprising the step of determining an amount of at least one species in a body fluid or a tissue homogenate sample which has been obtained from a patient, **characterized in that** the at least one species is selected from a group of species having molecular masses in a range of 3870 to 3895 Da, 4520 to 4545 Da, 4802 to 4836 Da, 4837 to 4871 Da and 7720 to 7790 Da, respectively.

2. The method of claim 1, **characterized in that** the at least one species is selected from a group of species having the molecular masses in the range of 4520 to 4545 Da, 4802 to 4836 Da, 4837 to 4871 Da and 7720 to 7790 Da, respectively.

3. The method of claim 1 or 2, **characterized in that** the amount of the at least one species is determined as a signal to noise ratio.

4. The method of any of the claims 1 to 3, **characterized in that** the sample is a cerebrospinal fluid or a brain homogenate.

5. The method of any of the claims 1 to 4, **characterized in that** the species having the molecular mass in the range of 4802 to 4836 Da is Aβ2-46 or Aβ1-45.

6. The method of claim 5, **characterized in that** the species having the molecular mass in the range of 4802 to 4836 Da is Aβ2-46.

7. The method of any of the claims 1 to 6, **characterized in that** the presence of the species having the molecular mass in the range of 4802 to 4836 Da is regarded as an indication of the presence of Alzheimer's disease.

8. The method of any of the claims 1 to 7, **characterized in that** the species having the molecular mass in the range of 4520 to 4545 Da is Aβ3-44.

9. The method of any of the claims 1 to 8, **characterized in that** the species having the molecular mass in the range of 4837 to 4871 Da is Aβ3-47.

10. The method of any of the claims 1 to 9, **characterized in that** a quotient of the amounts of the species having the molecular masses in the ranges of 4520 to 4545 Da and 4837 to 4871 Da is determined and compared with a cut off value, and that a quotient below the cut off value is regarded as an indication of the presence of Alzheimer's disease.

11. The method of claim 10, **characterized in that** the cut off value is in the range of 0.665 to 1.235, the amounts of the two species being the respective signal to noise ratios.

12. The method of any of the claims 1 to 11, **characterized in that** an amount below a cut off value of the species having the molecular mass in the range of 7720 to 7790 Da is regarded as an indication of the presence of Alzheimer's disease.

13. The method of claim 12, **characterized in that** a signal to noise ratio of the species having the molecular mass in the range of 7720 to 7790 Da which is below a cut off value in the range of 4.0 to 7.8 is regarded as an indication of the presence of Alzheimer's disease.

14. The method of any of the claims 1 to 13, **characterized in that** the amount of the at least one species is determined by surface enhanced laser desorption/ionization, the molecular masses being determined by time of flight mass spectrometry.

15. The method of any of the claims 1 to 14, **characterized in that** the sample is exposed to an antibody directed against Amyloid-beta (Aβ) peptides prior to determining the amount of the at least one species in a fraction of the sample bond to the antibody.

16. The method of any of the claims 1 to 15, **characterized in that** it further comprises the step of determining amounts of two further species in the sample which have molecular masses in the range of 4510 to 4519 Da and 4320 to 4340 Da, respectively.

17. The method of claim 16, **characterized in that** species having the molecular mass in the range of 4510 to 4519 Da is Aβ1-42 and that the species having the molecular mass in the range of 4320 to 4340 is Aβ1-40.

18. The method of any of the claims 16 and 17, **characterized in that** a quotient of two signal to noise ratios for the two further species having the molecular masses in the ranges of 4510 to 4519 and 4320 to 4340 is determined and compared with a cut off value in the range of 0.123 and 0.3, and that a quotient below the cut off value is regarded as an indication of the presence of Alzheimer's disease.
